# EUROPÄISCHE PATENTSCHRIFT

(11) **EP 2 775 256 B1**
(45) Veröffentlichungstag und Bekanntmachung des Hinweises auf die Patenterteilung: **10.07.2019**
(21) Anmeldenummer: 14450006.3
(22) Anmeldetag: 03.03.2014
(51) Int. Cl.: G01B 11/25, A61B 5/107, G06T 1/00

(54) **Verfahren zum optischen Erfassen der dreidimensionalen Geometrie von Objekten**
Method for optically detecting the three-dimensional geometry of objects
Procédé de détection optique de la géométrie tridimensionnelle d'objets

(30) Priorität: 07.03.2013 DE 102013102279
(43) Veröffentlichungstag der Anmeldung: 10.09.2014
(73) Patentinhaber: a.tron3d GmbH, 9020 Klagenfurt am Wörthersee (AT)
(72) Erfinder: Koinig,Horst, 9020 Klagenfurt (AT); Jesenko, Jürgen, 9584 Finkenstein (AT); Angerer, Helmut, 9020 Klagenfurt (AT)
(74) Vertreter: Beer & Partner Patentanwälte KG

(56) Entgegenhaltungen:
- WO-A1-2012/030357
- WO-A2-2007/084647

## Beschreibung

Die Erfindung betrifft ein Verfahren zum optischen Erfassen der dreidimensionalen Geometrie von Objekten, insbesondere Zähnen, mit einem Scanner, der einen Scankopf aufweist, wobei im Zuge des Scannens die erfasste dreidimensionale Geometrie in einem virtuellen dreidimensionalen Raum notiert wird. Insbesondere im Bereich dentaler Behandlungen sind viele Systeme zum optischen Erfassen der dreidimensionalen Geometrie von Objekten bekannt (siehe z.B. WO 2012/030357 A1). Diese finden beispielsweise Anwendung beim Erstellen von Prothesen, Kronen, Inlays oder dergleichen, dienen der Unterstützung bei der Überwachung von kieferorthopädischen Behandlungen und/oder helfen ganz allgemein bei der Beobachtung bzw. Erfassung von intraoralen Strukturen. Der große Vorteil derartiger optischer Systeme liegt zum einen darin, dass sie weder invasiv, noch unangenehm sind, wie beispielsweise der in der herkömmlichen Zahnmedizin häufig verwendete Zahnabdruck, und dass sie auch keine potentielle Gefährdung des Patienten darstellen, wie es beispielsweise bei strahlungsbasierten Methoden, wie dem Röntgen, der Fall ist. Zum anderen liegen die Daten nach dem Erfassen in elektronischer Form vor und lassen sich einfach speichern, beispielsweise für spätere Vergleiche, oder auch übermitteln, beispielsweise von einem Zahnarzt zu einem zahntechnischen Labor.

Ein Problem, das sich bei optischen Verfahren zum Erfassen der dreidimensionalen Geometrie von Objekten, insbesondere Zähnen, immer wieder stellt, ist, dass im Mundraum vorhandene Weichteile, wie die Innenseite der Wangen oder die Zunge, unbeabsichtigt erfasst werden. Eine spätere Korrektur solcher fehlerhaften Aufnahmen gestaltet sich üblicherweise schwierig, da auch bei Systemen, die mehrere Aufnahmen des selben Bereiches vorsehen, die fehlerhaften Aufnahmen in die erfasste bzw. berechnete Geometrie mit einfließen und diese verfälschen. Systeme, die viele Einzelbereiche aneinander fügen ("stitching"), können derartige Fehlmessungen noch schlechter überwinden, da häufig kein Anknüpfungspunkt zwischen einzelnen Bereichen gefunden werden kann.

Aufgabe der Erfindung ist es daher, ein Verfahren zur Verfügung zu stellen, das dieses Problem vermindert.

Gelöst wird diese Aufgabe bei einem Verfahren der eingangs genannten Art gemäß dem unabhängigen Anspruch 1 dadurch, dass im Zuge des Scannens Positionen einer definierten Scannergeometrie des Scanners, insbesondere des Scankopfes, relativ zum erfassten Objekt notiert werden, dass daraus ein Bereich, in dem sich die definierte Scannergeometrie befindet bzw. befunden hat, ermittelt wird und dass der ermittelte Bereich im virtuellen Raum, beispielsweise als "leer", markiert wird.

Das grundlegende Prinzip der Erfindung basiert - vereinfacht gesagt - also darauf, dass dort, wo sich der Scanner befindet bzw. befunden hat, kein festes Objekt, wie ein Zahn oder beispielsweise auch ein Implantat, sein kann.

Dies gilt insbesondere auch dann, wenn in einem Bereich, in dem sich der Scanner befindet oder befunden hat, bereits Oberflächeninformationen notiert sind. Dies kommt vor allem bei solchen Oberflächeninformationen vor, die wie oben beschrieben von der Zunge oder den Wangeninnenseiten eines Patienten erfasst wurden. In diesem Fall "radiert" man also derartige fälschlicherweise erfassten Oberflächen einfach weg, indem sie den Scanner an die vorherige Position der Zunge bewegt oder mit dem Scanner bzw. dem Scannerkopf die Wange des Patienten zur Seite drückt, und somit die entsprechende Scannerposition entsprechend markiert.

Diese für die Bedienperson einfache und besonders intuitive Handlung stellt sich gemäß einer bevorzugten Durchführungsform des Verfahrens so dar, dass, wenn zusätzlich zu den zu erfassenden Objekten wenigstens ein bewegbares Hemmobjekt, beispielsweise eine Zunge, erfasst wurde, der Scanner an die Position des Hemmobjektes bewegt wird und mögliche vom Hemmobjekt erfasste Daten, beispielsweise als "leer", markiert werden.

Dabei ist "leer" lediglich als beispielhafte Bezeichnung zum besseren Verständnis zu verstehen, "unbesetzt", "freier Raum" oder dergleichen bzw. entsprechende digitale Markierungen oder Informationen können ebenso notiert werden. Wesentlich ist nur, dass der ermittelte Bereich markiert und so hinterlegt wird, dass sich dort keine der zu vermessenden Objekte befinden bzw. befinden können, sondern lediglich Hemmobjekte, deren Erfassen nicht gewünscht ist bzw. deren Erfassen eine potentielle Fehlerquelle darstellt.

Ein zusätzlicher großer Vorteil der Erfindung, neben der intuitiven Anwendung für Bedienpersonen, liegt darin, dass einmal markierte Bereiche nicht mehr Teil weiterer Berechnungen sein müssen. Nach hinreichend vielen Bewegungen des Scanners bzw. des Scannerkopfes können bereits große Bereiche innerhalb des virtuellen Raumes markiert sein und es werden Rechnerressourcen gespart, die anderweitig, beispielsweise für eine schnellere Berechnung eines virtuellen Modells des erfassten Objektes, verwendet werden können.

Gemäß einer weiteren bevorzugten Durchführungsform des Verfahrens wird die Scannergeometrie des Scanners, insbesondere des Scannerkopfes, vor dem Erfassen der dreidimensionalen Geometrie der Objekte definiert und die Scannergeometrie ist gleich groß wie oder kleiner als der Scanner bzw. Scannerkopf. Diese repräsentative bzw. definierte Scannergeometrie vollzieht dann im virtuellen Raum die Bewegungen des realen Scanners bzw. Scannerkopfes nach. Die definierte Scannergeometrie kleiner zu gestalten als den tatsächlichen Scanner hat dabei zwei Vorteile bzw. Ursachen. Zum einen ragen große Teile des Scannerhandstücks aus dem Mundraum hinaus und sind für das Erfassen nicht relevant bzw. würden im virtuellen Raum Platz benötigen und so dessen Gesamtgröße gegebenenfalls negativ beeinflussen. Zum anderen wird die tatsächliche Form des Scanners üblicherweise nach ergonomischen und optischen Gesichtspunkten gewählt und kann daher viele, aus rechnerischer Sicht unnötig komplizierte Rundungen und Formen aufweisen. Selbstverständlich kann auch die exakte Form des Scanners als Scannergeometrie definiert werden. Allerdings ist auch eine simplifizierte Form, beispielsweise ein innerhalb der tatsächlichen Form des Scanners bzw. Scannerkopfes liegender Quader, für das Verfahren ausreichend und im Hinblick auf die benötigte Rechenleistung sogar vorteilhaft.

Gemäß einer weiteren bevorzugten Durchführungsform des Verfahrens ist der virtuelle Raum ein n³-Voxelgrid. Diese spezielle Art der Notation hat sich für die Notation von erfassten dreidimensionalen Geometrien als besonders geeignet herausgestellt. Gemäß einer bevorzugten Weiterbildung der Erfindung ist es damit möglich, ganze Voxel des n³-Voxelgrids zu markieren.

In einer Weiterbildung der Erfindung kann das Markieren durch eine Eingabe annulliert werden. In einer zusätzlichen oder alternativen Durchführungsform der Erfindung können markierte Bereiche auch ganz oder teilweise, durch eine Eingabe, zurückgesetzt werden. Dies kann beispielsweise dann vorteilhaft sein, wenn sich der Scanner in einem Bereich befunden hat, in dem eine Prothese eingesetzt wird. Bei Systemen, die das Fortsetzen des optischen Erfassens nach Veränderungen (beispielsweise Präparationen oder das Einsetzen von Prothesen) auf der Basis der ursprünglichen Aufnahme ermöglichen, würden als "leer" markierte Bereiche, in denen sich bei der Wiederaufnahme zu erfassende Oberflächen befinden, sonst gar nicht oder falsch erfasst werden.

Gemäß einer weiteren bevorzugten Durchführungsform der Erfindung werden Bereiche, welche auf der Seite der Scannergeometrie liegen, die der erfassten Geometrie abgewandt ist, ebenfalls markiert. Diese Durchführungsform ist vergleichbar mit einer Durchführungsform, bei welcher die definierte Scannergeometrie über die Rückseite des realen Scannerkopfes hinaus ragt. Der Vorteil dieser Durchführungsform ist, dass so der Anteil der Bereiche, die nicht mehr in die Berechnung der Oberfläche einfließen und damit die Rechenressourcen nicht belasten, vergrößert wird, ohne dass der Scanner tatsächlich an jeder dieser Stellen gewesen sein muss. Nachteilig ist dabei allerdings, dass, sollte beispielsweise die Innenseite eines Kieferbogens erfasst werden, auch Bereiche markiert werden können die tatsächlich erfasst werden sollen, wie beispielsweise ein der Innenseite gegenüberliegender Kieferbogen. Gemäß einer vorteilhaften weiterbildung kann dieses zusätzliche Markieren auch während einer laufenden Aufnahme zu- und abgeschaltet werden.

Gemäß einer weiteren zusätzlichen oder alternativen Durchführungsform der Erfindung werden markierte Bereiche zu Bereichsgruppen zusammengefasst. Beispielsweise können bei einem n³-Voxelgrid Voxel, ähnlich wie beim einem bottom-up Octree, in Achter-, Vierundsechziger-, Fünfhunderzwölfergruppen und dergleichen zusammengefasst werden. Bei einem Octree kann beispielsweise so die Markierung auf höhreren Octree-Leveln erfolgen und benötigt entsprechend weniger Speicherplatz.

Generell kann das Markieren von Bereichen nicht nur der Schonung von Rechenressourcen dienen, sondern auch der verbesserten Speicherverwaltung.

Dementsprechend kann in einer Weiterbildung der Erfindung bei Systemen, die das Fortsetzen des optischen Erfassens auf der Basis der ursprünglichen Aufnahme ermöglichen, die Korrespondenzfindung, also das Ermitteln der Stelle, an der das Erfassen fortgesetzt werden soll, erleichtert werden. Zum einen können markierte Bereiche oder Bereichsgruppen bei der Korrespondenzsuche übersprungen werden, was erhebliche Rechenzeit erspart, zum anderen können die markierten Bereiche selbst zu einem Kriterium der Korrespondenzfindung werden. Das heißt, dass Korrespondenzen nur dann als gültig betrachtet werden, wenn nicht nur die ermittelte Oberfläche mit der Korrespondenz-Oberfläche (innerhalb gewisser Toleranzen) übereinstimmt, sondern sich der Scanner auch in einem markierten Bereich, gegebenenfalls vollständig, befindet.

Weitere bevorzugte und vorteilhafte Durchführungsformen des erfindungsgemäßen Verfahrens sind Gegenstand der übrigen Unteransprüche.

Eine nur beispielhafte Anwendung der erfindungsgemäßen Verfahrens wird im Folgenden anhand der Zeichnungen näher erläutert. Diese zeigen in stark vereinfachter Form:
- Fig. 1: zwei Dimensionen eines Voxelgrids mit einer Scannergeometrie, und einer fehlerhaft erfassten Oberfläche eines symbolisch dargestellten Zahnes und einer Zunge,
- Fig. 2: das Voxelgrid von Fig. 1 mit einem markierten Bereich und
- Fig. 3: eine schematische Skizze zum Erfassen einer Position einer Scannergeomatrie.

Ein virtueller Raum zum Notieren von optisch erfassten Oberflächengeometrien ist üblicherweise dreidimensional und es gibt eine Vielzahl von unterschiedlichen Notationsmethoden für die erfasste Oberflächengeometrie innerhalb des virtuellen Raumes. Das dargestellte Beispiel zeigt eine bevorzugte Durchführungsform der Erfindung, bei der die Obeflächengeometrie in einem n³-Voxelgrid notiert wird. Diese Darstellung ist lediglich beispielhaft und nicht limitierend zu verstehen. Die wesentlichen Merkmale der Erfindung lassen sich auch auf andere Formen der Notation anwenden. Zum besseren Verständnis ist nur ein kleiner Teil des Voxelgrids abgebildet. Das Voxelgrid selbst ist dabei sehr viel grober dargestellt, als es in der Realität der Fall ist. Ebenso werden der Vereinfachung halber nur zwei Dimensionen gezeigt. Anwendungen der Erfindung, beispielsweise in der Implantologie, bei denen der virtuelle Raum zweidimensional ist, sind ebenso denkbar.

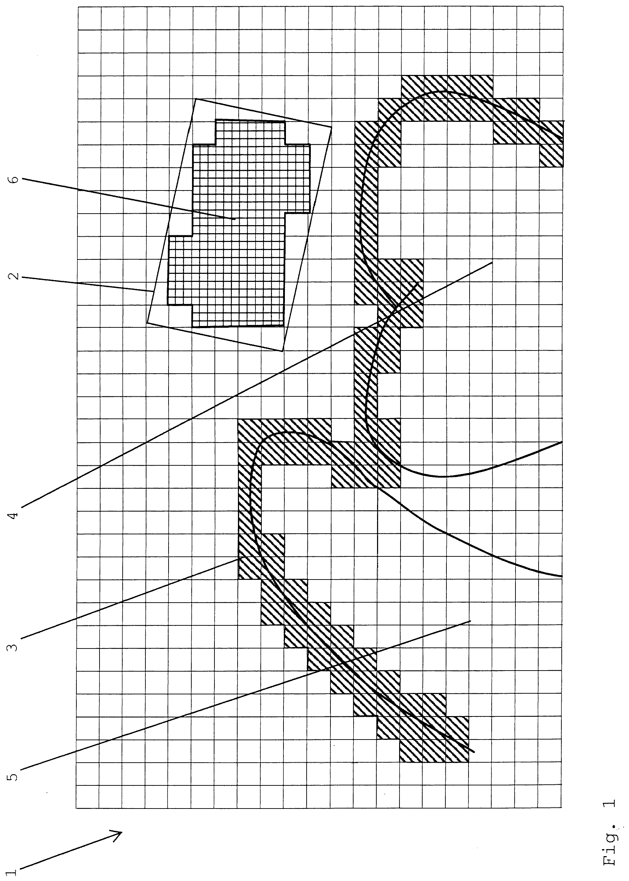

Die Fig. 1 zeigt zwei Dimensionen eines Voxelgrids 1 mit einer definierten Scannergeometrie 2 und einer fehlerhaft erfassten Oberfläche 3 eines symbolisch dargestellten Zahnes 4 und einer Zunge 5. Innerhalb der Scannergeometrie 2 ist ein Bereich 6 markiert. Dieser Bereich 6 umfasst dabei lediglich ganze Voxel, in denen sich die Scannergeometrie befindet. In alternativen Notationsarten kann selbstverständlich auch der gesamte Bereich, in welchem sich der Scanner befindet, notiert werden. Ebenso ist es auch möglich, Voxel, welche die Scannergeometrie nur teilweise enthalten, zu markieren. Die Wahl der Notation des Bereiches kann von einem Fachmann frei und gemäß seiner Art der optischen Erfassung der Oberflächengeometrie gewählt werden.

Die Scannergeometrie wird nun durch den virtuellen Raum, im dargestellten Beispiel also durch das Voxelgrid, bewegt. Die notwendigen Positionsdaten des realen Scanners lassen sich dabei im Wesentlichen auf zwei Arten gewinnen. Zum einen kann über einen Sensor im Scanner dessen Position, beispielsweise mit Bezug auf das Erdemagnetfeld, erfasst werden. Diese Methode ist allerdings unzuverlässig, da sich das Objekt auch bewegen kann und so der Scanner eine Position einnehmen kann, an welcher sich früher ein tatsächlich zu vermessendes Objekt befunden hat. Sehr viel zuverlässiger ist es dagegen, die Position des Scanners bzw. des Scannerkopfes im Zuge des optischen Erfassens der Oberflächengeometrie mit zu erfassen. Dabei wird die Position des Scanners bzw. Scannerkopfes relativ zur erfassten Oberfläche notiert.

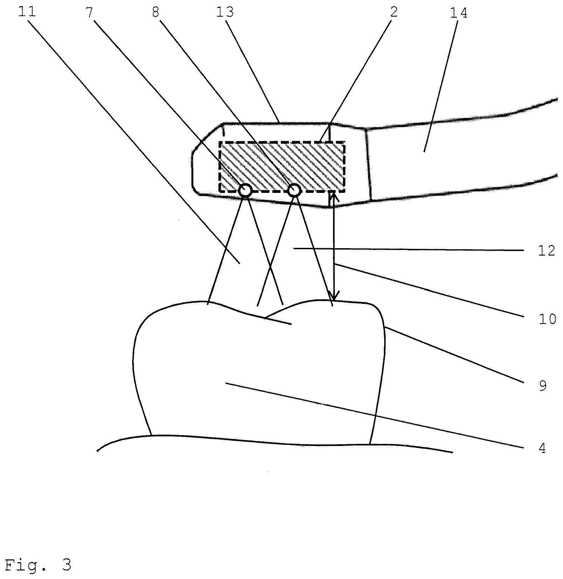

Das dieser Notation zugrundeliegende Prinzip ist in Fig. 3 skizziert. Alle Methoden zum optischen Erfassen von Oberflächengeometrien haben gemeinsam, dass eine Triangulation zwischen zwei Instrumenten 7, 8 und der realen Oberfläche 9 durchgeführt wird. Die Instrumente 7, 8 können dabei je nach System ein Projektor und ein Sensor sein oder zwei Sensoren. Dies kann durch zwei Aufnahmen eines Sensors aus verschiedenen bekannten Positionen oder durch zwei Sensoren mit einem bekannten räumlichen Verhältnis zueinander erfolgen. Im letzteren Fall handelt es sich um stereometrische Verfahren, wie sie gemäß einer bevorzugten Durchführungsform der Erfindung verwendet werden. Durch die Triangulation wird üblicherweise ein Abstand (dargestellt durch einen Pfeil 10) zwischen den Instrumenten 7, 8 und der Oberfläche 9 ermittelt. Die Instrumente 7, 8 haben üblicherweise kegelförmige Aufnahme- und/oder Projektionsbereiche 11, 12. Im dargestellten Beispiel sind die Instrumente 7, 8 zur einfacheren Erläuterung direkt in einem Scannerkopf 13 angeordnet. In der Realität befinden sich die Instrumente 7, 8 üblicherweise an anderen Orten innerhalb oder auch außerhalb des Scanners und die Projektion bzw. Aufnahme erfolgt unter Zuhilfenahme von Spiegeln, optischen Leitern und dergleichen. In diesem Fall stellen die kegelförmigen Bereiche die Aufnahme- und/oder Projektionsbereiche von Optiken von beispielsweise Kameras oder Projektoren dar. Systeme mit mehr Sensoren oder Projektoren sind ebenfalls bekannt. Die Erfindung kann auch auf solche Systeme angewendet werden.

Die Fig. 3 zeigt weiters eine beispielhaft definierte Scannergeometrie 2, welche innerhalb des Scannerkopfes 13 angeordnet ist. Im virtuellen Raum wird diese im dargestellten Beispiel bei jeder Aufnahme im ermittelten Abstand zur erfassten (virtuellen) Oberfläche positioniert. Bei anderen Anordnungen und Aufnahmemethoden kann die Position bezüglich der erfassten Oberfläche entsprechend des Scanneraufbaus vom Fachmann gewählt werden. Die Ausmaße der definierten Scannergeometrie 2 sind im dargestellten Beispiel deutlich kleiner gewählt als die Ausmaße des realen Scannerkopfes 13. So wird unter anderem vermieden, dass Messfehler zu einem "Ausradieren" gewünschter Oberflächeninformationen führen.

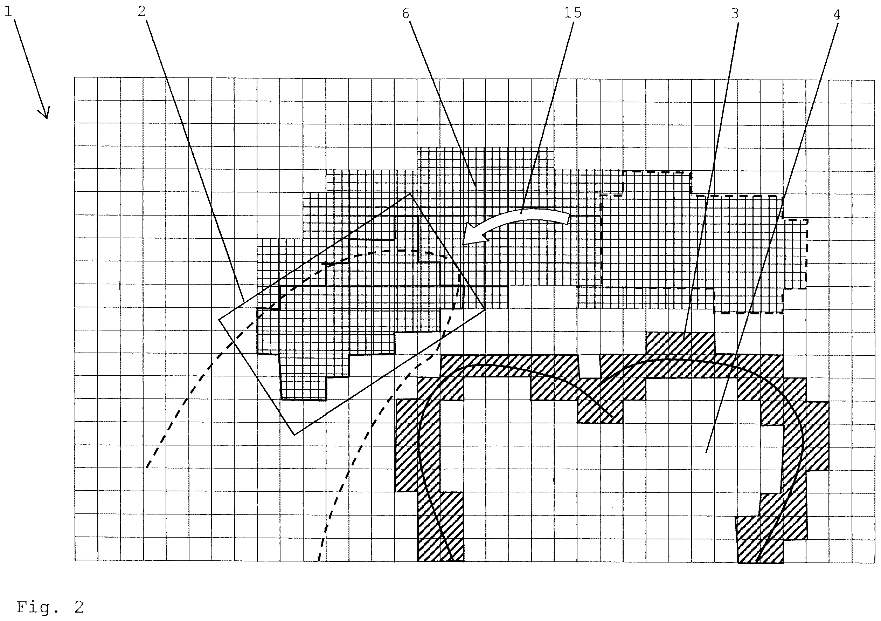

Die Fig. 2 zeigt das Voxelgrid von Fig. 1 mit einem markierten Bereich. Die Scannergeometrie 2 wird dabei, wie durch einen Pfeil 15 angedeutet, durch den virtuellen Raum bewegt. Der Bereich 6 mit den als "leer" markierten Voxeln vergrößert sich dabei. Die in Fig. 1 vom Hemmobjekt, d.h. von der Zunge 5, erfasste Oberfläche wird durch die Scannergeometrie 2 überschrieben bzw. markiert und fließt nicht mehr in Berechnungen der Oberflächengeometrie mit ein.

## Patentansprüche

1. Verfahren zum optischen Erfassen der dreidimensionalen Geometrie von Objekten, insbesondere Zähnen (4), mit einem Scanner (14), der einen Scankopf (13) aufweist, wobei im Zuge des Scannens die erfasste dreidimensionale Geometrie in einem virtuellen dreidimensionalen Raum (1) notiert wird, **dadurch gekennzeichnet, dass** im Zuge des Scannens ermittelte Positionen einer definierten Scannergeometrie (2) des Scanners (14), insbesondere des Scankopfes (13), relativ zum erfassten Objekt (4) notiert werden, dass daraus ein Bereich (6), in dem sich die definierte Scannergeometrie (2) befindet bzw. befunden hat, ermittelt wird und dass der ermittelte Bereich (6) im virtuellen Raum (1), beispielsweise als "leer", markiert wird.

2. Verfahren nach Anspruch 1 **dadurch gekennzeichnet, dass**, wenn zusätzlich zu den zu erfassenden Objekten (4) wenigstens ein bewegbares Hemmobjekt, beispielsweise eine Zunge (5), erfasst wurde, der Scanner (14) an die Position des Hemmobjektes (5) bewegt wird und dass mögliche vom Hemmobjekt (5) erfasste Daten, beispielsweise als "leer", markiert werden.

3. Verfahren nach Anspruch 1 oder 2, **dadurch gekennzeichnet, dass** die Scannergeometrie (2) des Scanners (14), insbesondere des Scannerkopfes (13), vor dem Erfassen definiert wird und dass die Scannergeometrie (2) kleiner als oder gleich groß wie der Scanner (14) bzw. Scannerkopf (13) ist.

4. Verfahren nach einem der Ansprüche 1 bis 3, **dadurch gekennzeichnet, dass** das Erfassen stereometrisch erfolgt.

5. Verfähren nach einem der Ansprüche 1 bis 4, **dadurch gekennzeichnet, dass** der virtuelle dreidimensionale Raum (1) ein n³-Voxelgrid ist.

6. Verfahren nach Anspruch 5, **dadurch gekennzeichnet, dass** Voxel des n³-Voxelgrids markiert werden, beispielsweise als "leer" , wenn sie den ermittelten Bereich (6) wenigstens teilweise enthalten.

7. Verfahren nach einem der Ansprüche 1 bis 6, **dadurch gekennzeichnet, dass** das Markieren durch eine Eingabe annulliert wird.

8. Verfahren nach einem der Ansprüche 1 bis 7, **dadurch gekennzeichnet**, das markierte Bereiche zurückgesetzt werden.

9. Verfahren nach einem der Ansprüche 5 bis 8, **dadurch gekennzeichnet, dass** Voxel zurückgesetzt werden.

10. Verfahren nach einem der Ansprüche 1 bis 9, **dadurch gekennzeichnet, dass** Bereiche, welche auf der Seite der Scannergeometrie (2) liegen, die der erfassten Geometrie (3) abgewandt ist, ebenfalls markiert werden.

11. Verfahren nach einem der Ansprüche 1 bis 10, **dadurch gekennzeichnet, dass** markierte Bereiche zu Bereichsgruppen zusammengefasst werden.

## Claims

1. Method for optical acquisition of the three-dimensional geometry of objects, in particular teeth (4), with a scanner (14), which has a scanning head (13), whereby the three-dimensional geometry that is acquired in the course of scanning is noted in a virtual three-dimensional space (1), **characterized in that**, positions of a defined scanner geometry (2) of the scanner (14), in particular of the scanning head (13), that are acquired in the course of scanning are noted relative to the object (4) that is being acquired, **in that**, from that an area (6) in which the defined scanner geometry (2) is or was located is determined, and **in that** the determined area (6) is marked, for example as "empty," in the virtual space (1).

2. Method according to claim 1, wherein if in addition to the objects (4) to be acquired at least one movable obstructing object, for example a tongue (5), was acquired, the scanner (14) is moved to the position of the obstructing object (5) and wherein possible data acquired from the obstructing object (5) are marked, for example as "empty."

3. Method according to claim 1 or 2, wherein the scanner geometry (2) of the scanner (14), in particular of the scanning head (13), is defined before acquisition, and wherein the scanner geometry (2) is smaller than or the same size as the scanner (14) or scanning head (13).

4. Method according to one of claims 1 to 3, wherein the acquisition is done stereometrically.

5. Method according to one of claims 1 to 4, wherein the virtual three-dimensional space (1) is an n³-voxel grid.

6. Method according to claim 5, wherein voxels of the n³-voxel grid are marked, for example as "empty," when they at least partially contain the determined area (6).

7. Method according to one of claims 1 to 6, wherein the marking is cancelled by an input.

8. Method according to one of claims 1 to 7, wherein the marked areas are reset.

9. Method according to one of claims 5 to 8, wherein voxels are reset.

10. Method according to one of claims 1 to 9, wherein areas that lie on the side of the scanner geometry (2), which is facing away from the acquired geometry (3), are also marked.

11. Method according to one of claims 1 to 10, wherein marked areas are clustered into area groups.

## Revendications

1. Procédé, destiné à la détection optique de la géométrie tridimensionnelle d'objets, notamment de dents (4), à l'aide d'un scanner (14), qui comporte une tête de scanner (13), dans lequel au cours du scannage, la géométrie tridimensionnelle détectée étant notée dans un espace virtuel (1) tridimensionnel, **caractérisé en ce qu'**on note des positions déterminées au cours du scannage d'une géométrie de scanner (2) définie du scanner (14), notamment de la tête de scanner (13) par rapport à l'objet (4) détecté, **en ce qu'**on détermine à partir de celles-ci une zone (6), dans laquelle se trouve ou s'est trouvée la géométrie de scanner (2) définie et **en ce qu'**on marque la zone (6) déterminée, par exemple comme étant « vide », dans l'espace virtuel (1).

2. Procédé selon la revendication 1 **caractérisé en ce que**, si en supplément de l'objet à détecter (4), au moins un objet inhibiteur mobile, par exemple une langue (5), a été détecté, on déplace le scanner (14) sur la position de l'objet inhibiteur (5) et **en ce qu'**on marque des données éventuelles détectées par l'objet inhibiteur (5), par exemple comme étant « vides ».

3. Procédé selon la revendication 1 ou 2, **caractérisé en ce qu'**on définit avant la détection la géométrie de scanner (2) du scanner (14), notamment de la tête de scanner (13), et **en ce que** la géométrie de scanner (2) est de taille inférieure ou égale à celle du scanner (14) ou de la tête de scanner (13) .

4. Procédé selon l'une quelconque des revendications 1 à 3, **caractérisé en ce que** la détection s'effectue stéréométriquement.

5. Procédé selon l'une quelconque des revendications 1 à 4, **caractérisé en ce que** l'espace virtuel (1) tridimensionnel est une grille de voxels n³.

6. Procédé selon la revendication 5, **caractérisé en ce qu'**on marque, par exemple comme étant vides, des voxels de la grille de voxels n³ s'ils contiennent au moins partiellement la zone (6) déterminée.

7. Procédé selon l'une quelconque des revendications 1 à 6, **caractérisé en ce qu'**on annule le marquage par une entrée.

8. Procédé selon l'une quelconque des revendications 1 à 7, **caractérisé en ce qu'**on réinitialise des zones marquées.

9. Procédé selon l'une quelconque des revendications 5 à 8, **caractérisé en ce qu'**on réinitialise des voxels.

10. Procédé selon l'une quelconque des revendications 1 à 9, **caractérisé en ce qu'**on aussi marque des zones, lesquelles se situent du côté de la géométrie de scanner (2) qui est opposé à la géométrie (3) détectée.

11. Procédé selon l'une quelconque des revendications 1 à 10, **caractérisé en ce qu'**on groupe des zones marquées en groupes de zones.
